# EUROPÄISCHE PATENTSCHRIFT

(11) **EP 4 376 700 B1**
(45) Veröffentlichungstag und Bekanntmachung des Hinweises auf die Patenterteilung: **21.05.2025**
(21) Anmeldenummer: 22744451.0
(22) Anmeldetag: 06.07.2022
(51) Int. Cl.: A61B 3/12, A61B 3/13, G02B 21/00, G02B 7/02, A61B 90/70, G02B 25/00, G02B 27/00, A61F 9/00, A61B 3/125

(54) **KONTAKTLOSES VISUALISIERUNGSSYSTEM FÜR EIN OPERATIONSMIKROSKOP FÜR DIE AUGENCHIRURGIE**
CONTACTLESS VISUALIZATION SYSTEM FOR A SURGICAL MICROSCOPE FOR EYE SURGERY
SYSTÈME DE VISUALISATION SANS CONTACT POUR UN MICROSCOPE CHIRURGICAL POUR CHIRURGIE OCULAIRE

(30) Priorität: 26.07.2021 DE 102021119297
(43) Veröffentlichungstag der Anmeldung: 05.06.2024
(73) Patentinhaber: Carl Zeiss Meditec AG, 07745 Jena (DE)
(72) Erfinder: ESSIG, Jonathan, 73447 Oberkochen (DE); BEDER, Christian, 73447 Oberkochen (DE)
(74) Vertreter: Patentanwälte Geyer, Fehners & Partner mbB
(86) Internationale Anmeldenummer: PCT/EP2022/068719
(87) Internationale Veröffentlichungsnummer: WO 2023/006366

(56) Entgegenhaltungen:
- EP-B1- 2 613 688
- WO-A1-2015/001200
- WO-A1-99/20171
- US-B2- 10 765 315

## Beschreibung

Die vorliegende Erfindung betrifft ein kontaktloses Visualisierungssystem für ein Operationsmikroskop für die Augenchirurgie mit den Merkmalen des Oberbegriffs des Anspruches 1.

Zur Ophthalmochirurgie des Hinterabschnitts des Auges werden gerne sogenannte kontaktlose Visualisierungssysteme eingesetzt, die die bisherige Visualisierung mittels eines auf dem Patientenauge aufgesetzten Kontaktglases zurückgedrängt haben. Diese Systeme arbeiten mit sogenannten Ophthalmoskopierlupen, die nahe über dem Patientenauge platziert werden und ein reelles (wenn auch seiten- und höhenverkehrtes) Bild des Augenhintergrunds in eine Zwischenbildebene liefern, die dann wiederum mit einem Operationsmikroskop betrachtet werden kann.

Durch die nahe Platzierung der Ophthalmoskopierlupe am Patientenauge engen sie den Arbeitsraum des Chirurgen ein. Dies ist gerade bei mehrlinsigen Ophthalmoskopierlupen (die beispielsweise zwei Linsen aufweisen) eine Schwierigkeit, da die hier notwendige Fassung für die mehreren Linsen den Arbeitsraum für den Chirurgen weiter einengen. Dies führt zu einer unerwünschten Beeinträchtigung des Operationsvorgangs.

Die US 10 765 315 B2 beschreibt ein kontaktloses Visualisierungssystem für ein Operationsmikroskop für die Augenchirurgie mit den Merkmalen des Oberbegriffs des Anspruches 1. Die WO 99/20171 A1 beschreibt ein indirektes Visualisierungssystem mit einer dem Auge zugewandten Kontaktlinse und einer davon beabstandeten weiteren Linse.

Ausgehend hiervon ist es Aufgabe der Erfindung, ein kontaktloses Visualisierungssystem der eingangs genannten Art so weiterzubilden, dass die genannten Schwierigkeiten möglichst vollständig überwunden werden können.

Die Erfindung ist in den unabhängigen Ansprüchen 1 und 15 definiert. Vorteilhafte Ausgestaltungen sind in den abhängigen Ansprüchen angegeben.

Da die Wandung gleichzeitig als Fassung für die zweite Linse dient, muss sie aus Gründen der Stabilität gewisse mechanische Mindestgrößen und -dicken aufweisen. Die notwendige Stabilität wird erfindungsgemäß beibehalten, da lediglich die mindestens eine Aussparung, in der der Rand der zweiten Linse freiliegt, vorgesehen ist, um einem Chirurgen mehr Arbeitsraum bereitzustellen.

Die Aussparung kann sich in der Wandung vom freien Ende in Richtung zur ersten Linse erstrecken, wobei sich die Aussparung bevorzugt nicht bis zur oberen Seite der ersten Linse erstreckt, sondern davor endet. Somit ist selbst im Bereich der Aussparung noch eine gewisse Höhe der Wandung vorgesehen, was aus Stabilitätsgründen vorteilhaft ist.

Die Aussparung kann sich in Richtung zur ersten Linse hin verjüngen.

Es kann sich um genau eine Aussparung, in der der Rand der zweiten Linse freiliegt, oder um mehrere Aussparungen, in der der Rand der zweiten Linse freiliegt, handeln. Die Wandung kann so ausgebildet sein, dass keine weitere Öffnung oder Aussparung vorgesehen ist. Alternativ ist es jedoch durchaus möglich, dass in der Wandung noch eine oder mehrere weitere Öffnungen und/oder Aussparungen ausgebildet sind.

Da die Wandung gleichzeitig als Fassung für die zweite Linse dient, muss sie aus Gründen der Stabilität gewisse mechanische Mindestgrößen und -dicken aufweisen. Es ist bevorzugt, wenn die Wandung in Umfangsrichtung durchgängig ausgebildet ist. Das heißt, vorzugsweise sind die Aussparung(en) und ggf. die weitere(n) Öffnung(en) derart angeordnet, dass die Wandung in Umfangsrichtung nicht unterbrochen bzw. vollständig unterbrochen ist. Bevorzugt ist die Wandung daher so ausgebildet, dass in jeder Position in Umfangsrichtung die Wandung mindestens einen Abschnitt aufweist, der sich in der Richtung von der ersten zur zweiten Linse erstreckt. Anders gesagt kann die Wandung so ausgebildet sein, dass die Wandung in Umfangsrichtung in sich geschlossen ist bzw. dass in Umfangsrichtung mindestens eine gedachte, in sich geschlossene Kurve auf der Wandung verläuft.

Insbesondere sind zwei Aussparungen vorgesehen, die in Umfangsrichtung bevorzugt um 80° bis 140°, insbesondere 90° bis 130° und besonders bevorzugt um 120° beabstandet sind. Diese Beabstandung bezieht sich bevorzugt auf die Mitte der jeweiligen Aussparung in Umfangsrichtung. Die Breite der Aussparung am freien Ende in Umfangsrichtung kann 20° bis 70°, insbesondere 30° bis 60° oder 30° bis 50° sowie besonders bevorzugt 35° bis 45° aufweisen. So kann die Breite beispielsweise 40° betragen.

Die Ophthalmoskopierlupe kann einen Halteranschluss aufweisen, mit dem die Ophthalmoskopierlupe mechanisch mit dem Operationsmikroskop verbindbar ist, wobei der Halteranschluss mit der Fassung an einer Verbindungsstelle verbunden ist und die mindestens eine Aussparung um 90° bis 150° in Umfangsrichtung von der Verbindungsstelle beabstandet ist (bevorzugt bezogen auf die Mitte der Aussparung in Umfangsrichtung und auf die Mitte der Verbindungsstelle in Umfangsrichtung).

Ferner kann mindestens eine Durchgangsöffnung in der Wandung ausgebildet sein. Diese mindestens eine Durchgangsöffnung dient dazu, um eventuell im Zwischenraum zwischen den beiden Linsen vorhandene Flüssigkeit entfernern zu können. Dabei kann es sich genau um eine Durchgangsöffnung zur Flüssigkeitsentfernung oder um mehrere Durchgangsöffnungen zur Flüssigkeitsentfernung handeln.

Somit wird einerseits die notwendige mechanische Stabilität gewährleistet und andererseits eine Flüssigkeitsentfernung sichergestellt.

Die mindestens eine Durchgangsöffnung kann in Umfangsrichtung eine Breite von 20° bis 70°, insbesondere von 30° bis 60° oder 30° bis 50° sowie besonders bevorzugt von 35° bis 45° aufweisen. Die Breite kann z.B. 40° betragen.

Insbesondere kann die Wandung zusammen mit der ersten Linse als einstückiges Teil ausgebildet sein. Besonders bevorzugt ist es, dass es sich um ein Spritzgussteil handelt. Damit wird die Möglichkeit geschaffen, die Ophthalmoskopierlupe als Einmalprodukt bzw. Einwegartikel auszubilden und gleichzeitig die notwendige optische Qualität sicherzustellen. Mittels der Fassung kann gewährleistet werden, dass die zweite Linse an der optisch vorgegebenen Position relativ zur ersten Linse positioniert ist. Die einstückige Ausbildung der Wandung mit der ersten Linse ist bevorzugt als Stoffschluss realisiert. Es ist jedoch auch eine Realisierung mittels Formschluss möglich.

Insbesondere kann die zweite Linse konzentrisch zur ersten Linse angeordnet sein.

Die mindestens eine Durchgangsöffnung ist bevorzugt näher an der ersten Linse als an der zweiten Linse ausgebildet. Insbesondere kann die mindestens eine Durchgangsöffnung so ausgebildet sein, dass ihr unterer Rand direkt an die Grenzfläche der ersten Linse stößt, die der zweiten Linse zugewandt ist. Dieses Anstoßen des unteren Rands an die Grenzfläche der ersten Linse ist bevorzugt so ausgebildet, dass dadurch keine Vertiefung vorgesehen ist. Beispielsweise kann der untere Rand senkrecht zur optischen Achse der ersten Linse verlaufen.

Bei dem erfindungsgemäßen Visualisierungssystem können mehrere Durchgangsöffnungen in der Wandung ausgebildet und in Umfangsrichtung voneinander beabstandet sein. Somit kann die mindestens eine Durchgangsöffnung mehrere Durchgangsöffnungen umfassen Dabei kann es sich um zwei, drei, vier, fünf oder mehr Durchgangsöffnungen handeln.

Es können z.B. drei Durchgangsöffnungen vorgesehen sein, die jeweils um 120° in Umfangsrichtung voneinander beabstandet sind. Diese Beabstandung bezieht sich bevorzugt auf die Mitte der jeweiligen Durchgangsöffnung in Umfangsrichtung.

Die erste Linse und/oder die Wandung können aus Kunststoff hergestellt sein. Neben der ersten Linse kann auch die zweite Linse aus Kunststoff hergestellt sein. Die zweite Linse kann einen Halteranschluss aufweisen, der integral mit der zweiten Linse ausgebildet sein kann. Insbesondere kann der Halteranschluss zusammen mit der zweiten Linse durch ein Zweikomponenten-Spritzgussverfahren hergestellt sein. Die integrale Ausbildung der zweiten Linse mit dem Halteranschluss ist bevorzugt als Formschluss realisiert. Es ist jedoch auch eine Realisierung mittels Formschluss möglich.

Die Fassung kann als Schnappverschluss, Klemmverbindung oder elastische Klemmhalterung ausgebildet sein. Dazu kann die Fassung mehrere elastische Klemmfinger (beispielsweise drei, die in Umfangsrichtung voneinander beabstandet sind, beispielsweise um 120° bezogen auf die Mitte des jeweiligen Klemmfingers in Umfangsrichtung) aufweisen, die z.B. jeweils eine Klemmnut umfassen. Die zweite Linse kann entsprechende Fassungsbereiche aufweisen, die in die Klemmnuten einstehen. Ferner kann die Fassung entsprechende Fassungsbereiche aufweisen, die Anschlagflächen für die zweite Linse bzw. für die Fassungsbereiche der zweiten Linse aufweisen.

Es ist jedoch auch jede andere Art der Halterung mittels der Fassung möglich, wie z.B. ein Schraubverschluss, ein Bajonettverschluss, etc.

Es ist bevorzugt, wenn die mindestens eine Aussparung und die mindestens eine Durchgangsöffnung in Umfangsrichtung versetzt zueinander angeordnet sind. Insbesondere können die mindestens eine Aussparung und die mindestens eine Durchgangsöffnung so angeordnet sein, dass sie in Umfangsrichtung jeweils voneinander beabstandet sind. Der Abstand kann beispielsweise 60° betragen (bezogen auf die Mitte der jeweiligen Aussparung in Umfangsrichtung und auf die Mitte der jeweiligen Durchgangsöffnung in Umfangsrichtung).

Ferner kann die erste Linse zusammen mit der Wandung eine n-zählige Drehsymmetrie aufweisen, wenn n Durchgangsöffnungen und/oder n Aussparungen vorgesehen sind, wobei n eine ganze Zahl ≥ 1 und ≤ 6 ist. Besonders bevorzugt ist n = 3.

Das kontaktlose Visualisierungssystem kann ferner einen Ophthalmoskopierlupenträger aufweisen, mit dem die Ophthalmoskopierlupe mechanisch verbunden ist. Mittels des Ophthalmoskopierlupenträgers kann die Ophthalmoskopierlupe im Abbildungsstrahlengang des Operationsmikroskops positioniert und aus dem Abbildungsstrahlengang bewegt werden. Dazu kann der Ophthalmoskopierlupenträger ferner mechanisch mit dem Operationsmikroskop verbunden sein. Ferner kann der Ophthalmoskopierlupenträger so ausgebildet sein, dass eine Rotationsbewegung der Ophthalmoskopierlupe um die optische Achse des Operationsmikroskops möglich ist. Damit kann ein Benutzer die Ophthalmoskopierlupe in eine von ihm gewünschte Rotationsstellung bringen, die für ihn z.B. bei der Verwendung der Ophthalmoskopierlupe vorteilhaft ist.

Des Weiteren kann die mechanische Verbindung der Ophthalmoskopierlupe mit dem Ophthalmoskopierlupenträger eine lösbare Verbindung sein, so dass die Ophthalmoskopierlupe austauschbar ist. Dies ist besonders vorteilhaft, wenn die Ophthalmoskopierlupe als Einwegartikel ausgebildet ist.

Das erfindungsgemäße Operationsmikroskop für die Augenchirurgie kann insbesondere als 3D-Operationsmikroskop ausgebildet sein.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine schematische Ansicht eines Ausführungsbeispiels des kontaktlosen Visualisierungssystems 1 in Verbindung mit einem Operationsmikroskop 2;
- Fig. 2: eine perspektivische Darstellung der Ophthalmoskopierlupe 3 von Fig. 1;
- Fig. 3: eine perspektivische Schnittdarstellung der Ophthalmoskopierlupe 3 von Fig. 2;
- Fig. 4: eine Ansicht von unten auf die erste Linse 27₁ der Ophthalmoskopierlupe 3 von Fig. 1;
- Fig. 5: eine perspektivische Darstellung der ersten Line 27₁ mit der Wandung 30 der Ophthalmoskopierlupe 3 von Fig. 1;
- Fig. 6: eine perspektivische Darstellung eines weiteren Ausführungsbeispiels der Ophthalmoskopierlupe 3 von Fig. 1, und
- Fig. 7: eine perspektivische Darstellung eines weiteren Ausführungsbeispiels der Ophthalmoskopierlupe 3 von Fig. 1.

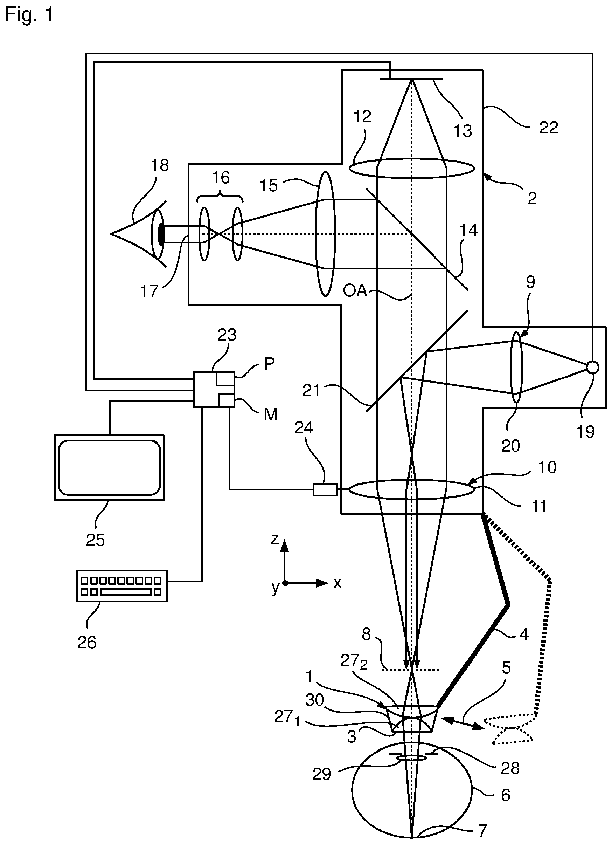

Bei dem in Fig. 1 gezeigten Ausführungsbeispiel eines kontaktlosen Visualisierungssystems 1 ist dieses zusammen mit einem Operationsmikroskop 2 für die Augenchirurgie gezeigt. Das kontaktlose Visualisierungssystem 1 umfasst eine Ophthalmoskopierlupe 3 sowie einen Ophthalmoskopierlupenträger 4, mit dem die Ophthalmoskopierlupe 3 in einen Abbildungsstrahlengang des Operationsmikroskops 2 eingeschoben (bzw. positioniert) und aus dem Abbildungsstrahlengang herausgeschoben (bzw. entfernt) werden kann, wie durch den Doppelpfeil 5 in Fig. 1 schematisch dargestellt ist. Die Position der Ophthalmoskopierlupe 3 außerhalb des Abbildungsstrahlengangs ist in Fig. 1 gestrichelt dargestellt.

Wie ferner Fig. 1 zu entnehmen ist, kann mittels des Ophthalmoskopierlupenträgers 4 die Ophthalmoskopierlupe 3 nahe über einem Patientenauge 6 platziert werden, so dass ein reelles sowie höhen- und seitenverkehrtes Bild des Augenhintergrunds 7 des Patientenauges 6 in eine Zwischenbildebene 8 abgebildet wird. Dieses reelle sowie höhen- und seitenverkehrte Bild des Augenhintergrunds 7 wird dann mit dem Operationsmikroskop 2 betrachtet.

Das Operationsmikroskop 2 kann eine Beleuchtungseinheit 9 zum Beleuchten des Patientenauges 6 (hier z.B. des Augenhintergrunds 7) sowie eine Abbildungsoptik 10 zum vergrößerten Abbilden des beleuchteten Patientenauges 6 und hier auch zum vergrößerten Abbilden des Bildes in der Zwischenbildebene 8 aufweisen. Das Patientenauge 6 mit Augenpupille 28 und Augenlinse 29 ist hier nur schematisch dargestellt.

Die in Fig. 1 schematisch dargestellte Abbildungsoptik 10 umfasst ein Objektiv 11 und eine erste Tubusoptik 12, um den beleuchteten Bereich des Patientenauges 6 auf einen Bildsensor 13 abzubilden. Des Weiteren umfasst die Abbildungsoptik 10 einen ersten Strahlteiler 14, der zwischen dem Objektiv 11 und der ersten Tubusoptik 12 angeordnet ist, sowie eine dem ersten Strahlteiler 14 nachgeordnete zweite Tubusoptik 15 und eine der zweiten Tubusoptik 15 nachgeordnete Okularoptik 16, so dass ein optischer Einblick 17 bereitgestellt ist, wie durch das schematisch dargestellte Auge 18 eines Benutzers angedeutet ist.

Die Beleuchtungseinheit 9 umfasst eine Lichtquelle 19, eine der Lichtquelle 19 nachgeordnete Kollektoroptik 20, einen zwischen dem Objektiv 11 und der ersten Tubusoptik 12 angeordneten zweiten Strahlteiler 21 und das Objektiv 11. Das Licht der Lichtquelle 19 wird von der Kollektoroptik 20 so fokussiert und mittels des zweiten Strahlteilers 21 so in den Strahlengang zwischen dem zweiten Strahlteiler 21 und dem Objektiv 11 eingekoppelt, dass der zu beleuchtende Bereich des Patientenauges 6 möglichst gleichmäßig beleuchtet wird, wie in Fig. 1 mit einem schematischen Strahlenverlauf bis zur Zwischenbildebene 8 angedeutet ist. Die Lichtquelle 19 kann beispielsweise als Halogenlampe, als Xenon-Entladungslampe, als LED oder als Laser ausgebildet sein.

Die bisher beschriebenen Elemente des Operationsmikroskops 2 sind in einem Gehäuse 22 angeordnet, wie in Fig. 1 schematisch dargestellt ist. Der Ophthalmoskopierlupenträger 4 ist mechanisch mit dem Gehäuse 22 verbunden, wobei zusätzlich zu den in Fig. 1 schematisch angedeuteten Ein- und Ausschieben der Ophthalmoskopierlupe 3 auch eine Revolvermechanik am Ophthalmoskopierlupenträger 4 ausgebildet sein kann, so dass auch eine Rotation der Ophthalmoskopierlupe 3 um die z-Achse und hier um die optische Achse OA des Abbildungsstrahlengangs des Operationsmikroskops 2 möglich ist.

Ferner umfasst das Operationsmikroskop 2 ein Steuergerät 23, das einen Prozessor P und einen Speicher M aufweist und z.B. mit der Lichtquelle 19, dem Bildsensor 13 und einem z-Trieb 24, mit dem das Objektiv 11 in der z-Richtung zur Fokussierung bewegt werden kann, verbunden ist.

Ferner kann ein Bildschirm 25 sowie eine Eingabeeinheit 26 mit dem Steuergerät 23 verbunden sein, wie schematisch in Fig. 1 dargestellt ist. Die Eingabeeinheit 26 ist hier nur schematisch als Tastatur dargestellt. Es sind auch sonstige Eingabeeinheiten, wie z.B. Fußschalter und ähnliches möglich.

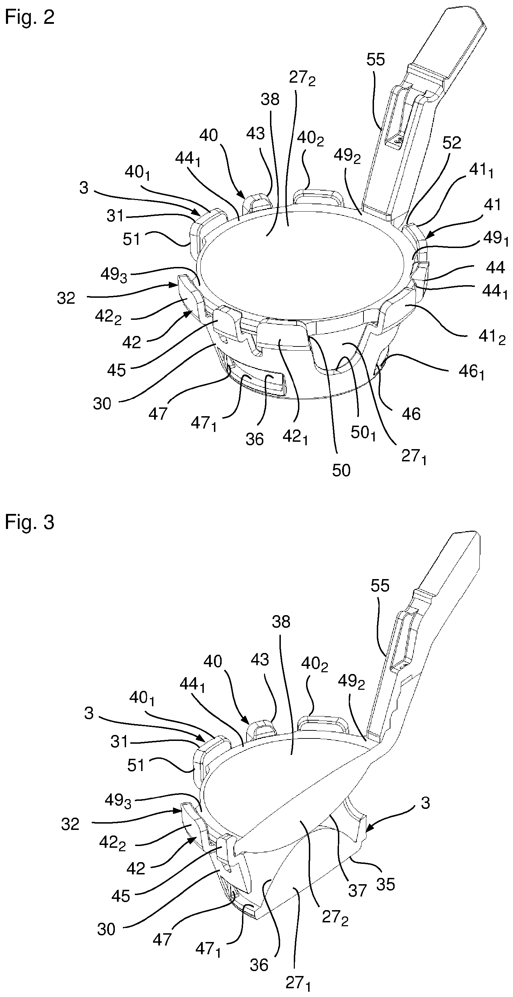

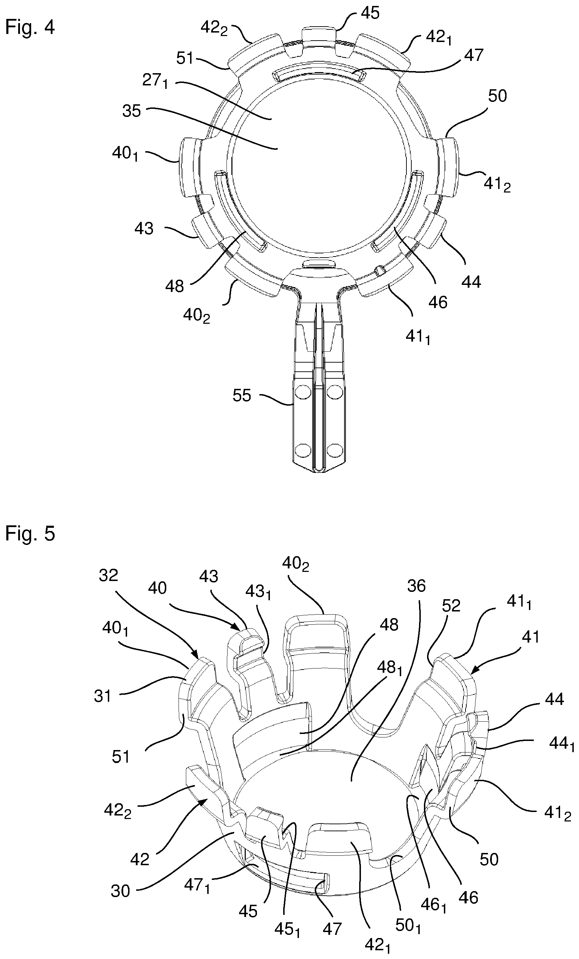

Die Ophthalmoskopierlupe 3 umfasst hier genau eine erste Kunststofflinse 27₁ mit einer ersten und zweiten Grenzfläche 35, 36 und eine zweite Kunststofflinse 27₂ mit einer ersten und zweiten Grenzfläche 37, 38, wobei eine sich in Richtung zur zweiten Linse 27₂ erstreckende Wandung 30 einstückig mit der ersten Kunststofflinse 27₁ ausgebildet ist. Das freie Ende 31 der Wandung 30 dient als Fassung 32, wie insbesondere in Verbindung mit Figuren 2-5 erkennbar ist. Die Grenzflächen 35-38 sind bevorzugt gekrümmt ausgebildet, wobei mindestens eine der Grenzflächen 35-38 asphärisch gekrümmt ist. Die verbleibende(n) Grenzfläche(n) ist bzw. sind bevorzugt sphärisch gekrümmt.

Die Fassung 32 umfasst einen ersten, einen zweiten und einen dritten Fassungsabschnitt 40, 41 und 42, die in Umfangsrichtung jeweils um 120° voneinander beabstandet sind. Jeder Fassungsabschnitt 40-42 weist einen ersten und zweiten Anschlagbereich 40₁, 40₂; 41₁, 41₂ sowie 42₁ und 42₂ auf, zwischen denen jeweils ein elastischer Klemmfinger 43, 44, 45 mit einer Klemmnut 43₁, 44₁, 45₁ angeordnet ist.

Die zweite Linse 27₂ weist drei Fassungsbereiche 49₁-49₃ (insbesondere in Figuren 2 und 3 erkennbar) auf, die in den Klemmnuten 43₁, 44₁ und 45₁ der elastischen Klemmfinger 43-45 eingeklemmt sind und an den Anschlagbereichen 40₁-40₃ so anliegen, dass die zweite Linse 27₂ konzentrisch zur ersten Linse 27₁ gehalten ist. Diese Art der Klemmverbindung zwischen der Fassung 32 und der zweiten Linse 27₂ kann auch als Schnappverbindung bezeichnet werden.

Die zweite Linse 27₂ weist noch einen Halteranschluss 55 auf, über den eine lösbare Verbindung mit dem Ophthalmoskopierlupenträger 4 möglich ist.

Da die Wandung 30 gleichzeitig als Fassung 32 für die zweite Linse 27₂ dient, muss sie aus Gründen der Stabilität gewisse mechanische Mindestgrößen und -dicken aufweisen. Dies führt dazu, dass die Wandung 30 im Wesentlichen geschlossen ausgebildet ist, so dass sich zwischen den beiden Linsen 27₁ und 27₂ zwar Flüssigkeit sammeln kann, die jedoch nicht wieder abfließen kann. Dies würde dazu führen, dass die optische Abbildungsqualität sehr stark leidet, wodurch unter Umständen nur noch eine teilweise scharfe Abbildung oder gar keine scharfe Abbildung mehr möglich ist.

Daher weist die Wandung 30 hier in ihrem unteren Bereich (und somit nahe an der ersten Linse 27₁) drei in Umfangsrichtung voneinander beabstandete Durchgangsöffnungen 46, 47 und 48 auf, die hier in Umfangsrichtung um 120° zueinander versetzt angeordnet sind. Die Breite jeder Durchgangsöffnung 46-48 in Umfangsrichtung kann z.B. 40° betragen. Aufgrund dieser Durchgangsöffnungen 46-48 ist es möglich, dass die unerwünschte Flüssigkeit entweder von selbst abfließt und/oder der Benutzer (beispielsweise der Chirurg) die Flüssigkeit bei Bedarf entfernt. Dazu kann beispielsweise ein entsprechendes, Flüssigkeit aufsaugendes Werkzeug, (wie z.B. ein Tupfer) an oder in die Durchgangsöffnung 46-48 gehalten werden.

Somit lässt sich unerwünschte Flüssigkeit einfach aus dem Zwischenraum zwischen den beiden Linsen 27₁ und 27₂ entfernen und die gewünschte optische Abbildungsqualität auf Dauer sicherstellen.

Wie insbesondere in Figur 3 ersichtlich ist, sind die Durchgangsöffnungen 46-48 bevorzugt so ausgebildet, dass ihr unterer Rand 46₁, 47₁, 48₁ an die obere Grenzfläche 36 der ersten Linse 27₁ stößt. Die obere Grenzfläche 36 ist die Grenzfläche der ersten Linse 27₁, die zur zweiten Linse 27₂ weist. Bevorzugt ist der untere Rand 46₁, 47₁, 48₁ der Durchgangsöffnung 46-48 so ausgebildet, dass keine Vertiefung zwischen dem unteren Rand 46₁, 47₁, 48₁ der Durchgangsöffnung 46-48 und der oberen Grenzfläche 36 erzeugt wird. Im hier vorliegenden Fall ist der untere Rand 46₁, 47₁, 48₁ beispielsweise so ausgebildet, dass er sich im Wesentlichen senkrecht zur optischen Achse der ersten Linse 27₁ erstreckt.

Ferner besteht die Problematik, dass aufgrund der Fassung 32 mehr Platz seitlich von der zweiten Linse 27₂ in Anspruch genommen wird im Vergleich zu bisherigen nahezu randlosen Ophthalmoskopierlupen mit nur einer einzelnen Linse. Dadurch wird der Arbeitsraum des Chirurgen eingeschränkt. Um die gewünschte Halterung der zweiten Linse 27₁ mittels der Fassung 32 bereitstellen zu können, sind gewisse mechanische Mindestgrößen und -dicken der Wandung 30 aus Gründen der Stabilität zwingend erforderlich. Jedoch wurde erkannt, dass es dazu nicht notwendig ist, dass die Fassung 32 den gesamten Rand der zweiten Linse 27₂ umfasst. Daher ist die Wandung 30 hier so ausgebildet, dass zwei Aussparungen 50 und 51 vorgesehen sind, in denen der Rand der zweiten Linse 27₂ freiliegt. Die zwei Aussparungen 50 und 51 sind in Umfangsrichtung um z.B. 120° voneinander beabstandet. Ferner ist noch eine dritte Aussparung 52 vorgesehen, um ausreichend Platz für den Halteranschluss 55 im Bereich der Fassung 32 bereitzustellen.

Für den Benutzer bzw. Chirurgen sind jedoch die Aussparungen 50 und 51 vorgesehen. Wenn man die Position des Halteranschlusses 55 als zwölf Uhr bezeichnet, sind die erste und zweite Aussparung 50 und 51 bevorzugt an den Positionen 4 Uhr und 8 Uhr vorgesehen, da dort die Hände des Chirurgen zu liegen kommen.

Wie insbesondere in Figuren 2-4 ersichtlich ist, erstreckt sich die erste und zweite Aussparung 50 und 51 vom freien Ende 31 in Richtung zur ersten Linse 27₁. In dem hier beschriebenen Ausführungsbeispiel erstrecken sich die Aussparungen 50-51 nicht bis zur oberen Seite 36 der ersten Linse 27₁, sondern enden davor, um aus Stabilitätsgründen noch eine gewisse Höhe der Wandung 30 im Bereich der Aussparungen 50 und 51 vorzusehen. Der untere Rad 50₁, 50₂ der Aussparungen 50 und 51 liegt somit oberhalb des unteren Randes 46₁, 47₁, 48₁ der Durchgangsöffnungen 46-48. Gleiches gilt für den unteren Rand 52₁ der Aussparung 52. Jede der beiden Aussparungen 50 und 51 weist am freien Ende 31 der Wandung 30 in Umfangsrichtung eine Ausdehnung von beispielsweise 40° auf. Der Abstand der Aussparungen 50 und 51 in Umfangsrichtung, der hier beispielsweise 120° beträgt, bezieht sich auf die Mitte der Aussparung 50, 51 am freien Ende 31 der Wandung 30. Die Aussparung 52 ist in Umfangsrichtung um 120° von den beiden Aussparungen 50 und 51 beabstandet.

Wie insbesondere in Figuren 2, 3 und 5 zu entnehmen ist, verjüngt sich die Ausdehnung (bzw. Breite) der Aussparung 50, 51 in Umfangsrichtung in eine Richtung von der zweiten Linse 27₂ zur ersten Linse 27₁. Man kann auch sagen, dass die Breite der Aussparungen 50, 51 in Richtung zum unteren Rand 50₁, 50₂ abnimmt bzw. dass sich die Aussparungen 50, 51 verjüngen.

Wie den Figuren 2-5 zu entnehmen ist, sind die Durchgangsöffnungen 46-48 und die Aussparungen 50-52 in Umfangsrichtung so angeordnet, dass jeweils abwechselnd eine Aussparung 50-52 und eine Durchgangsöffnung 46-48 vorgesehen ist. Sie sind in Umfangsrichtung z.B. jeweils um 60° zueinander versetzt angeordnet. In dieser Art und Weise kann an jeder Stelle der Wandung 30 eine gewünschte Mindesthöhe und somit eine gewisse Mindeststabilität bereitgestellt werden.

Insbesondere ist die Wandung 30 somit in Umfangsrichtung durchgängig ausgebildet. Die Wandung 30 ist daher in Umfangsrichtung nicht unterbrochen. Es gibt somit in Umfangsrichtung keine Position, an der sich nicht in der Richtung von der ersten zur zweiten Linse 27₁, 27₂ über mindestens eine gewisse Teilstrecke ein Teil der Wandung erstreckt. Anders gesagt, kann man sich eine in sich geschlossene Kurve denken, die auf der Wandung in Umfangsrichtung verläuft. Es gibt somit in Umfangsrichtung stets einen in sich geschlossenen Weg.

Die erste Linse 27₁ (mit der Wandung 30) ist hier in der Art symmetrisch ausgebildet, dass eine Drehung der ersten Linse 27₁ (mit der Wandung 30) um 120° um die optische Achse der ersten Linse 27₁ (mit der Wandung 30) auf sich selbst abbildet. Es liegt somit eine 3-zählige Drehsymmetrie vor.

Die Ophthalmoskopierlupe 3 kann insbesondere so ausgebildet sein, dass sie lösbar mit dem Ophthalmoskopierlupenträger 4 verbunden ist. In diesem Fall ist es beispielsweise bevorzugt, dass die Ophthalmoskopierlupe 3 als Einwegartikel ausgebildet ist und somit genau einmal verwendbar ist. Nach der Verwendung wird die Ophthalmoskopierlupe 3 entsorgt, wodurch insbesondere keine Probleme mit einer sonst notwendigen Reinigung und Desinfektion der Ophthalmoskopierlupe 3 auftreten. Die Ophthalmoskopierlupe 3 ist in diesem Fall bevorzugt aus Kunststoff hergestellt. Insbesondere kann sie ein Kunststoffspritzteil sein.

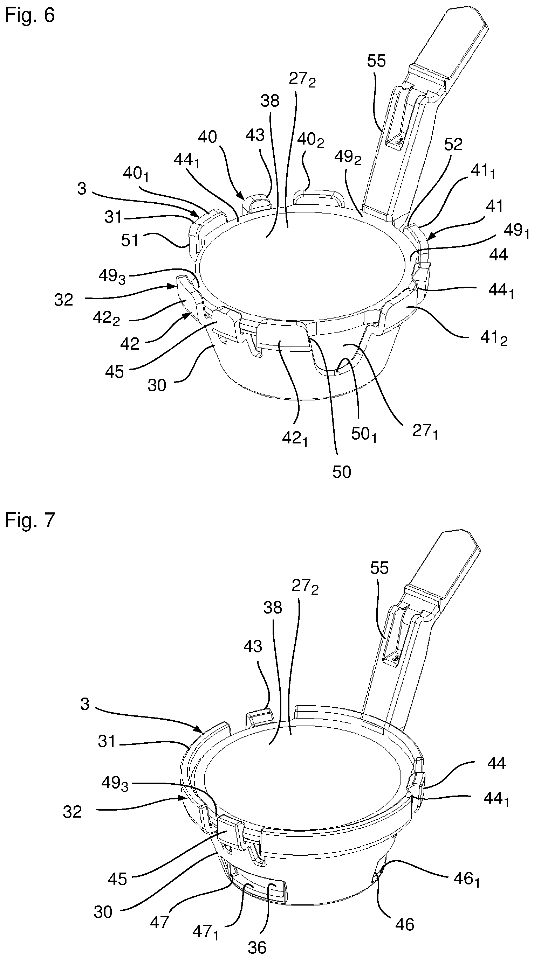

In Fig. 6 ist ein weiteres Ausführungsbeispiel eines kontaktlosen Visualisierungssystems 1 gezeigt, das zusammen mit dem Operationsmikroskop 2 gemäß Fig. 1 verwendet werden kann. Bei diesem Ausführungsbeispiel sind lediglich die Aussparungen 50, 51 und 52 vorgesehen. Die Wandung 30 weist jedoch keine Durchgangsöffnungen auf, wie sie bei dem Ausführungsbeispiel von Figuren 2-5 vorhanden sind.

Bei dem Ausführungsbeispiel gemäß Fig. 6 wird der Vorteil erreicht, dass dem Benutzer bzw. Chirurgen ausreichend Arbeitsraum auch im Bereich der zweiten Linse 27₂ bereitgestellt ist, da die Aussparungen 50 und 51 vorgesehen sind. Somit unterscheidet sich das Ausführungsbeispiel gemäß Fig. 6 von dem Ausführungsbeispiel gemäß Figuren 2-5 nur darin, dass keine Durchgangsöffnungen vorgesehen sind. Alle anderen Merkmale und Ausgestaltungen der Ausführungsformen von Figuren 2-5 sind jedoch vorhanden.

In Fig. 7 ist ein weiteres Ausführungsbeispiel eines kontaktlosen Visualisierungssystems 1 gezeigt, das zusammen mit dem Operationsmikroskop 2 gemäß Fig. 1 verwendet werden kann. Bei diesem Ausführungsbeispiel weist die Ophthalmoskopierlupe 3 nur die Durchgangsöffnungen 46-48 auf. Nicht jedoch sind die Aussparungen 50-52 vorgesehen. Ansonsten sind alle Merkmale des Ausführungsbeispiels gemäß Figur 2-5 in gleicher Weise verwirklicht. Somit weist das Ausführungsbeispiel gemäß Fig. 7 den Vorteil auf, das Flüssigkeit, die sich in dem Raum zwischen den beiden Linsen 27₁ und 27₂ ansammeln kann, entweder von selbst abfließt und/oder vom Benutzer bei Bedarf entfernt werden kann.

## Patentansprüche

1. Kontaktloses Visualisierungssystem (1) für ein Operationsmikroskop (2) für die Augenchirurgie,
wobei das kontaktlose Visualisierungssystem (1) eine Ophthalmoskopierlupe (3) aufweist, die vor einem Patientenauge (6) positionierbar ist und ein reelles sowie höhen- und seitenverkehrtes Bild des Augenhintergrunds (7) des Patientenauges (6) in eine Zwischenbildebene (8) liefert, die mit dem Operationsmikroskop (2) betrachtbar ist,
wobei die Ophthalmoskopierlupe (3) eine erste und eine zweite Linse (27₁, 27₂) aufweist, wobei die erste Linse (27₁) im vor dem Patientenauge (6) positionierten Zustand näher am Patientenauge (6) ist als die zweite Linse (27₂), und
wobei sich von der ersten Linse (27₁) zur zweiten Linse (27₂) eine Wandung (30) erstreckt, deren freies Ende (31) als Fassung (32) ausgebildet ist, in der die zweite Linse (27₂) gefasst ist, und
**dadurch gekennzeichnet, dass**
die Fassung (32) mindestens eine Aussparung (50, 51) aufweist, in der der Rand der zweiten Linse (27₂) freiliegt, um einem Chirurgen Arbeitsraum bereitzustellen.

2. Kontaktloses Visualisierungssystem (1) nach Anspruch 1,
**dadurch gekennzeichnet, dass** die Wandung (30) in Umfangsrichtung durchgängig ausgebildet ist.

3. Kontaktloses Visualisierungssystem (1) nach Anspruch 1 oder 2,
**dadurch gekennzeichnet**, die Ophthalmoskopierlupe (3) eine Halteranschluss (55) aufweist, mit dem die Ophthalmoskopierlupe (3) mechanisch mit dem Operationsmikroskop (2) verbindbar ist,
wobei der Halteranschluss (55) mit der Fassung (32) an einer Verbindungsstelle verbunden ist und die Mitte der mindestens einen Aussparung (50, 51) um 90° bis 150° in Umfangsrichtung von der Mitte der Verbindungsstelle in Umfangsrichtung beabstandet ist.

4. Kontaktloses Visualisierungssystem (1) nach einem der obigen Ansprüche,
**dadurch gekennzeichnet, dass** die mindestens eine Aussparung (50, 51) zwei Aussparungen (50, 51) umfasst, welche in der Fassung (32) ausgebildet sind und deren Mitten in Umfangsrichtung um 60° bis 180° voneinander beabstandet sind.

5. Kontaktloses Visualisierungssystem (1) nach einem der obigen Ansprüche,
**dadurch gekennzeichnet, dass** sich die mindestens eine Aussparung (50, 51) am freien Ende (31) in Umfangsrichtung um 20° bis 70° erstreckt.

6. Kontaktloses Visualisierungssystem (1) nach einem der obigen Ansprüche,
**dadurch gekennzeichnet, dass** sich die mindestens eine Aussparung (50, 51) in der Wandung (30) vom freien Ende in Richtung zur ersten Linse (27₁) so erstreckt, dass die mindestens eine Aussparung (50, 51) oberhalb der ersten Linse (27₁) endet und somit noch eine vorbestimmte Höhe der Wandung (30) unterhalb der mindestens einen Aussparung (50, 51) vorhanden ist.

7. Kontaktloses Visualisierungssystem (1) nach einem der obigen Ansprüche,
**dadurch gekennzeichnet, dass** die Wandung (30) zusammen mit der ersten Linse (27₁) als ein einstückiges Teil ausgebildet ist.

8. Kontaktloses Visualisierungssystem (1) nach einem der obigen Ansprüche,
**dadurch gekennzeichnet, dass** in der Wandung (30) mindestens eine Durchgangsöffnung (47, 48, 49) ausgebildet ist, um eventuell im Zwischenraum zwischen erster und zweiter Linse (27₁, 27₂) vorhandene Flüssigkeit entfernen zu können.

9. Kontaktloses Visualisierungssystem (1) nach Anspruch 8,
**dadurch gekennzeichnet, dass** die mindestens eine Durchgangsöffnung (47, 48, 49) näher an der ersten Linse (27₁) als an der zweiten Linse (27₂) ausgebildet ist.

10. Kontaktloses Visualisierungssystem (1) nach Anspruch 8 oder 9,
**dadurch gekennzeichnet, dass** mehrere Durchgangsöffnungen (47, 48, 49) in der Wandung (30) ausgebildet und in Umfangsrichtung voneinander beabstandet sind.

11. Kontaktloses Visualisierungssystem (1) nach einem der Ansprüche 8 bis 10,
**dadurch gekennzeichnet, dass** die erste Linse (27₁) eine zur zweiten Linse (27₂) weisende Grenzfläche (36) aufweist, die gekrümmt ausgebildet ist, wobei die mindestens eine Durchgangsöffnung (47, 48, 49) einen unteren Rand (47₁, 48₁, 49₁) aufweist, der an die Grenzfläche (36) der ersten Linse (27₁) stößt.

12. Kontaktloses Visualisierungssystem (1) nach einem der Ansprüche 8 bis 11,
**dadurch gekennzeichnet, dass** die mindestens eine Durchgangsöffnung (47, 48, 49) in Umfangsrichtung versetzt zur mindestens einen Aussparung (50, 51) angeordnet ist.

13. Kontaktloses Visualisierungssystem (1) nach einem der obigen Ansprüche,
**dadurch gekennzeichnet,**
**dass** die Fassung (32) als elastische Klemmhalterung (43, 44, 45) ausgebildet ist.

14. Kontaktloses Visualisierungssystem (1) nach einem der obigen Ansprüche,
**dadurch gekennzeichnet, dass** ein Ophthalmoskopierlupenträger (4) vorgesehen ist, mit dem die Ophthalmoskopierlupe (3) mechanisch verbunden ist und der dazu dient, die Ophthalmoskopierlupe (3) vor dem Patientenauge (6) zu positionieren,
wobei bevorzugt die Ophthalmoskopierlupe (3) lösbar mit dem Ophthalmoskopierlupenträger (4) verbunden ist.

15. Operationsmikroskop (2) für die Augenchirurgie,
wobei das Operationsmikroskop (2) ein kontaktloses Visualisierungssystem (1) nach einem der obigen Ansprüche aufweist.

## Claims

1. Contactless visualization system (1) for a surgical microscope (2) for eye surgery,
the contactless visualization system (1) comprising an ophthalmic loupe (3) which is positionable in front of a patient's eye (6) and supplies a real and vertically and laterally inverted image of the eye fundus (7) of the patient's eye (6) in an intermediate image plane (8) that is observable by the surgical microscope (2),
with the ophthalmic loupe (3) comprising a first and a second lens element (27₁, 27₂), with, in the state positioned in front of the patient's eye (6), the first lens element (27₁) being closer to the patient's eye (6) than the second lens element (27₂), and
with a wall (30) extending from the first lens element (27₁) to the second lens element (27₂), the free end (31) of said wall being in the form of a mount (32) in which the second lens element (27₂) is held, and **characterized in that**
the mount (32) comprises at least one cutout (50, 51), in which the edge of the second lens element (27₂) is exposed in order to give a surgeon operating space.

2. Contactless visualization system (1) according to Claim 1,
**characterized in that** the wall (30) is formed throughout in the circumferential direction.

3. Contactless visualization system (1) according to Claim 1 or 2,
**characterized in that** the ophthalmic loupe (3) comprises a holder connector (55), by means of which the ophthalmic loupe (3) is mechanically connectable to the surgical microscope (2),
wherein the holder connector (55) is connected to the mount (32) at a connection site and the center of the at least one cutout (50, 51) is spaced apart from the center of the connection site in the circumferential direction by 90° to 150° in the circumferential direction.

4. Contactless visualization system (1) according to any of the preceding claims,
**characterized in that** the at least one cutout (50, 51) comprises two cutouts (50, 51) which are formed in the mount (32) and the centers of which are spaced apart from one another by 60° to 180° in the circumferential direction.

5. Contactless visualization system (1) according to any of the preceding claims,
**characterized in that**, at the free end (31), the at least one cutout (50, 51) extends through 20° to 70° in the circumferential direction.

6. Contactless visualization system (1) according to any of the preceding claims,
**characterized in that** the at least one cutout (50, 51) extends in the wall (30) from the free end in the direction of the first lens element (27₁) such that the at least one cutout (50, 51) ends above of the first lens element (27₁) and hence a predetermined height of the wall (30) still is present below the at least one cutout (50, 51).

7. Contactless visualization system (1) according to any of the preceding claims,
**characterized in that** the wall (30) is formed as a one-piece part together with the first lens element (27₁).

8. Contactless visualization system (1) according to any of the preceding claims,
**characterized in that** at least one passage opening (47, 48, 49) is formed in the wall (30) in order to be able to remove liquid possibly present in the intermediate space between the first and second lens element (27₁, 27₂) .

9. Contactless visualization system (1) according to Claim 8,
**characterized in that** the at least one passage opening (47, 48, 49) is formed closer to the first lens element (27₁) than the second lens element (27₂).

10. Contactless visualization system (1) according to Claim 8 or 9,
**characterized in that** a plurality of passage openings (47, 48, 49) are formed in the wall (30) and spaced apart from one another in the circumferential direction.

11. Contactless visualization system (1) according to any of Claims 8 to 10,
**characterized in that** the first lens element (27₁) comprises a boundary surface (36) which faces the second lens element (27₂) and has a curved form, with the at least one passage opening (47, 48, 49) comprising a lower edge (47₁, 48₁, 49₁) which borders on the boundary surface (36) of the first lens element (27₁).

12. Contactless visualization system (1) according to any of Claims 8 to 11,
**characterized in that** the at least one passage opening (47, 48, 49) is arranged offset with respect to the at least one cutout (50, 51) in the circumferential direction.

13. Contactless visualization system (1) according to any of the preceding claims,
**characterized**
**in that** the mount (32) is in the form of an elastic clamping holder (43, 44, 45).

14. Contactless visualization system (1) according to any of the preceding claims,
**characterized in that** an ophthalmic loupe support (4) is provided and the ophthalmic loupe (3) is mechanically connected thereto, said ophthalmic loupe support serving to position the ophthalmic loupe (3) in front of the patient's eye (6),
wherein preferably the ophthalmic loupe (3) is detachably connected to the ophthalmic loupe support (4).

15. Surgical microscope (2) for eye surgery,
wherein the surgical microscope (2) comprises a contactless visualization system (1) according to any of the preceding claims.

## Revendications

1. Système de visualisation sans contact (1) pour un microscope chirurgical (2) pour la chirurgie oculaire, le système de visualisation sans contact (1) présentant une loupe d'ophtalmoscopie (3) qui peut être positionnée devant l'œil (6) d'un patient et qui fournit une image réelle ainsi qu'une image inversée en hauteur et en largeur du fond d'œil (7) de l'œil (6) du patient dans un plan d'image intermédiaire (8) qui peut être observé avec le microscope chirurgical (2),
la loupe d'ophtalmoscopie (3) présentant une première et une deuxième lentille (27₁, 27₂), la première lentille (27₁) étant plus proche de l'œil (6) du patient que la deuxième lentille (27₂) dans l'état positionné devant l'œil (6) du patient, et
une paroi (30) s'étendant de la première lentille (27₁) à la deuxième lentille (27₂), dont l'extrémité libre (31) est réalisée sous forme de monture (32) dans laquelle la deuxième lentille (27₂) est enchâssée, et
**caractérisé en ce que**
la monture (32) présente au moins un évidement (50, 51) dans lequel le bord de la deuxième lentille (27₂) est exposé pour fournir un espace de travail à un chirurgien.

2. Système de visualisation sans contact (1) selon la revendication 1,
**caractérisé en ce que** la paroi (30) est réalisée sous forme continue dans la direction circonférentielle.

3. Système de visualisation sans contact (1) selon la revendication 1 ou 2,
**caractérisé en ce que** la loupe d'ophtalmoscopie (3) présente un raccord de soutien (55) avec lequel la loupe d'ophtalmoscopie (3) peut être reliée mécaniquement au microscope chirurgical (2),
le raccord de soutien (55) étant relié à la monture (32) au niveau d'un point de liaison, et le centre de l'au moins un évidement (50, 51) étant espacé de 90° à 150° dans la direction circonférentielle du centre du point de liaison dans la direction circonférentielle.

4. Système de visualisation sans contact (1) selon l'une quelconque des revendications précédentes,
**caractérisé en ce que** l'au moins un évidement (50, 51) comprend deux évidements (50, 51) qui sont réalisés dans la monture (32) et dont les centres sont espacés de 60° à 180° dans la direction circonférentielle.

5. Système de visualisation sans contact (1) selon l'une quelconque des revendications précédentes,
**caractérisé en ce que** l'au moins un évidement (50, 51) à l'extrémité libre (31) s'étend dans la direction circonférentielle de 20° à 70°.

6. Système de visualisation sans contact (1) selon l'une quelconque des revendications précédentes,
**caractérisé en ce que** l'au moins un évidement (50, 51) dans la paroi (30) s'étend depuis l'extrémité libre en direction de la première lentille (27₁) de telle sorte que l'au moins un évidement (50, 51) se termine au-dessus de la première lentille (27₁) et qu'il reste donc une hauteur prédéterminée de la paroi (30) en dessous de l'au moins un évidement (50, 51).

7. Système de visualisation sans contact (1) selon l'une quelconque des revendications précédentes,
**caractérisé en ce que** la paroi (30) est réalisée en tant que pièce d'un seul tenant conjointement avec la première lentille (27₁).

8. Système de visualisation sans contact (1) selon l'une quelconque des revendications précédentes,
**caractérisé en ce qu'**au moins une ouverture de passage (47, 48, 49) est réalisée dans la paroi (30) pour permettre d'éliminer le liquide éventuellement présent dans l'espace entre les première et deuxième lentilles (27₁, 27₂).

9. Système de visualisation sans contact (1) selon la revendication 8,
**caractérisé en ce que** l'au moins une ouverture de passage (47, 48, 49) est réalisée plus près de la première lentille (27₁) que de la deuxième lentille (27₂).

10. Système de visualisation sans contact (1) selon la revendication 8 ou 9,
**caractérisé en ce que** plusieurs ouvertures de passage (47, 48, 49) sont réalisées dans la paroi (30) et sont espacées les unes des autres dans la direction circonférentielle.

11. Système de visualisation sans contact (1) selon l'une quelconque des revendications 8 à 10,
**caractérisé en ce que** la première lentille (27₁) présente une interface (36), réalisée sous forme incurvée faisant face à la deuxième, lentille (27₂), l'au moins une ouverture de passage (47, 48, 49) présentant un bord inférieur (47₁, 48₁, 49₁) qui vient buter contre l'interface (36) de la première lentille (27₁).

12. Système de visualisation sans contact (1) selon l'une quelconque des revendications 8 à 11,
**caractérisé en ce que** l'au moins une ouverture de passage (47, 48, 49) est agencée en décalage dans la direction circonférentielle par rapport à l'au moins un évidement (50, 51).

13. Système de visualisation sans contact (1) selon l'une quelconque des revendications précédentes,
**caractérisé en ce que**
la monture (32) est réalisée sous forme de soutien de serrage élastique (43, 44, 45).

14. Système de visualisation sans contact (1) selon l'une quelconque des revendications précédentes,
**caractérisé en ce qu'**il est prévu un support de loupe d'ophtalmoscopie (4) auquel la loupe d'ophtalmoscopie (3) est reliée mécaniquement et qui sert à positionner la loupe d'ophtalmoscopie (3) devant l'œil (6) du patient,
de préférence, la loupe d'ophtalmoscopie (3) étant reliée de manière amovible au support de loupe d'ophtalmoscopie (4).

15. Microscope opératoire (2) pour la chirurgie oculaire,
le microscope chirurgical (2) présentant un système de visualisation sans contact (1) selon l'une quelconque des revendications précédentes.
